## Europäisches Patentamt

## European Patent Office

## Office européen des brevets

(11) Veröffentlichungsnummer: **0 130 319**
A2

(12) **EUROPÄISCHE PATENTANMELDUNG**

(21) Anmeldenummer: 84105345.7

(22) Anmeldetag: 11.05.84

(51) Int. Cl.⁴: **C 07 D 301/32,** C 07 D 303/04, A 61 L 2/20

(30) Priorität: 25.06.83 DE 3322910
23.03.84 DE 3410711

(43) Veröffentlichungstag der Anmeldung: 09.01.85
Patentblatt 85/2

(84) Benannte Vertragsstaaten: **AT BE CH DE FR GB IT LI LU NL SE**

(71) Anmelder: **Leybold-Heraeus GmbH, Bonner Strasse 504 Postfach 51 07 60, D-5000 Köln 51 (DE)**

(72) Erfinder: **Amlinger, Heinrich, Johann-Schmidt-Strasse 12, D-6361 Niddatal 2 (DE)**

(74) Vertreter: **Leineweber, Jürgen, Dipl.-Phys., Bonner Strasse 498 Postfach 51 07 60, D-5000 Köln 51 (DE)**

(54) Verfahren und Vorrichtung zur Reinigung von Äthylenoxid oder einem Gemisch aus Äthylenoxid und einem fluorierten Chlorkohlenwasserstoff.

(57) Zur Reinigung von Äthylenoxid oder einem Gemisch aus Äthylenoxid und einem fluorierten Chlorkohlenwasserstoff wird vorgeschlagen, das Gas zu trocknen und bei einem oberhalb des Verflüssigungsdruckes von Luft liegenden Druck zu verflüssigen; zur Trocknung des Gases dient ein Adsorber (23) mit Molekularsieb, Silicagel, Alugel oder dergleichen; zur Verflüssigung des Gases wird ein ölfrei arbeitender Kompressor (32) verwendet.

- i -

Verfahren und Vorrichtung zur Reinigung von Äthylenoxid oder einem Gemisch aus Äthylenoxid und einem anwendungsspezifischen Zusatz

Äthylenoxid ist ein sehr toxisches und schon mit geringer Luftverunreinigung explosibles Gas, das u. a. zu Sterilisationszwecken in der Medizin Verwendung findet. Zur Veränderung seiner physikalischen Eigenschaften wird es häufig mit anwendungsspezifischen Zusätzen versetzt. So ist es z. B. bekannt, Äthylenoxid zur Verminderung seiner Explosivität mit einem fluorierten Chlorkohlenwasserstoff (vorzugsweise Dichlordifluormethan) zu vermischen. Andere Zusätze sind z. B. Kohlendioxid oder Methylformiat. Nach der Erfüllung seines Zweckes, also z. B. der Sterilisation, wird das Gas bzw. Gasgemisch abgeblasen bzw. abgefackelt. Infolge der toxischen Wirkung des Äthylenoxids und z. B. des fluorierten Chlorkohlenwasserstoffs oder der anderen Zusätze stellt das Abblasen bzw. Abfackeln eine starke Umweltbelastung dar.

Der vorliegenden Erfindung liegt die Aufgabe zugrunde, ein Verfahren vorzuschlagen, mit dem das Äthylenoxid oder das Gemisch aus Äthylenoxid und den anwendungsspezifischen Zusätzen bei wirtschaftlich vertretbarem Aufwand gereinigt und damit zur Wiederverwendung zurückgewonnen werden kann.

Erfindungsgemäß wird diese Aufgabe dadurch gelöst, daß das Äthylenoxid bzw. das Gemisch aus Äthylenoxid und anwendungsspezifischem Zusatz zunächst getrocknet und danach bei einem oberhalb des Verflüssigungsdruckes von Luft liegenden Druck verflüssigt wird. Durch diese Maßnahme erfolgt eine Trennung des Äthylenoxids bzw. des Gemisches aus Äthylenoxid und anwendungsspezifischem Zusatz

- 2 -

einerseits von Sauerstoff, Stickstoff und weiteren gasförmigen Verunreinigungen ähnlicher Art andererseits. In flüssiger Phase kann das Gas abgezogen werden. Nach einem Verdampfungsschritt steht es gereinigt dem jeweiligen Verwendungszweck wieder zur Verfügung.

Die Verflüssigung kann durch Druckerhöhung und/oder Temperaturerniedrigung erzielt werden. Wesentlich ist dabei, daß diese Werte so gewählt werden, daß die unerwünschten Verunreinigungen gasförmig bleiben. Bei Drücken im Atmosphärendruckbereich müssen die Temperaturen unterhalb 20$^\circ$ C (bei reinem Äthylenoxid) bzw. -30$^\circ$ C (bei Gemischen) liegen. Bei Temperaturen im Zimmertemperaturbereich (ca. 20$^\circ$ C) dürfen die jeweils gewählten Drücke ca.1,5 bar (bei reinem Äthylenoxid) bzw. ca. 5 bar (bei Gemischen) nicht unterschreiten. Die Arbeitstemperaturen können im Prinzip zwischen -193$^\circ$ C und 40$^\circ$ C liegen, vorzugsweise zwischen -80$^\circ$ C und 20$^\circ$ C. Dementsprechend sind Drücke zwischen 16 bar (und mehr) und 100 mbar möglich.

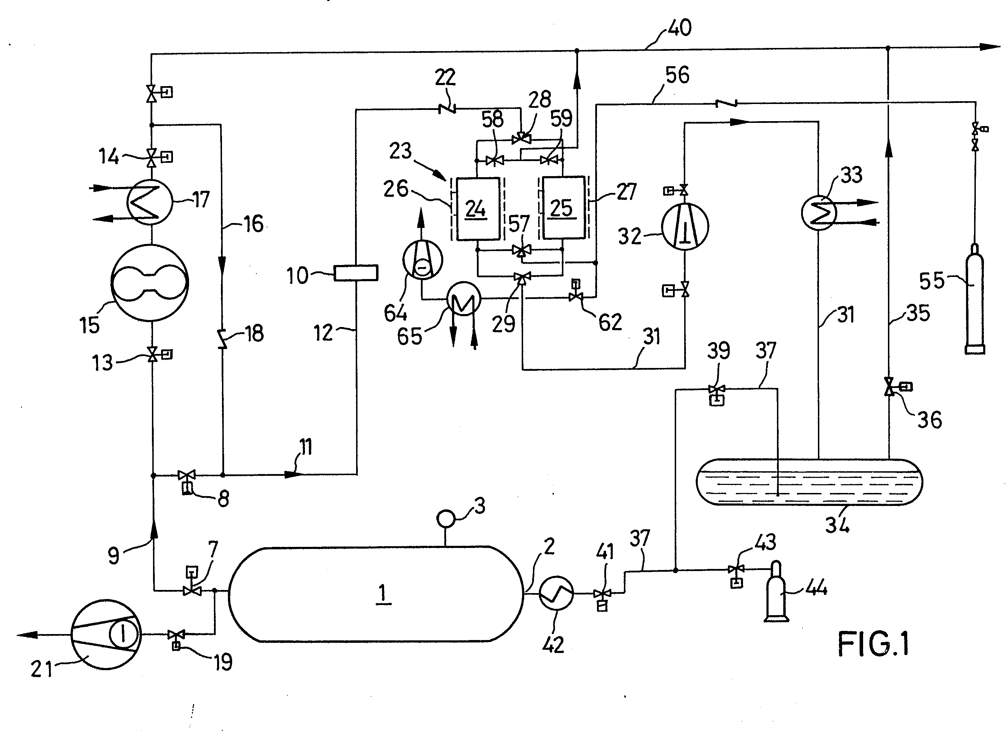

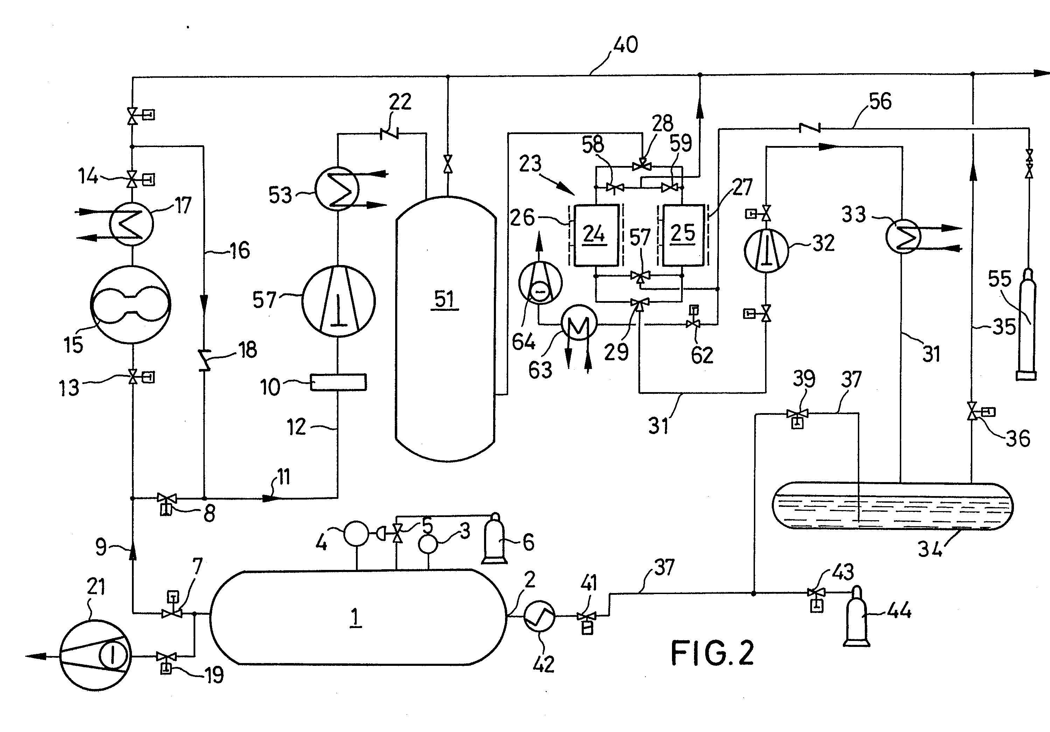

Weitere Vorteile und Einzelheiten der Erfindung sollen anhand der Figuren 1 und 2 erläutert werden. Es zeigen:

Fig. 1 eine Sterilisationsanlage, bei der Äthylenoxid bei erhöhtem Druck gereinigt und zurückgewonnen wird,

Fig. 2 eine entsprechende Anlage, die mit einem Gemisch aus Äthylenoxid und Dichlordifluormethan (z. B. 12 % Äthylenoxid, 88 % Dichlordifluormethan) arbeitet und

Fig. 3 eine Sterilisationsanlage, bei der das Arbeitsgas bei erniedrigter Temperatur gereinigt und zurückgewonnen wird.

In den Figuren ist der Behälter der Sterilisationsanlage, in dem das Arbeitsgas benutzt wird, die Sterilisationskammer, mit 1 bezeichnet. Dieser wird bei 2 gereinigtes bzw. zurückgewonnenes Gas zugeführt. Der Druck im Behälter 1 wird mittels eines Drucküberwachungsgerätes 3 kontrolliert. Bei der Anlage nach Fig. 2 ist zusätzlich eine Einrichtung 4 zur Überwachung der Gaszusammensetzung vorgesehen. In Abhängigkeit von dieser Einrichtung wird bei Bedarf über das Ventil 5 der fehlende Gasanteil - in der Regel Äthylenoxid - aus dem Gasreservoir 6 zugeführt.

In der Sterilisationskammer 1 liegt das Arbeitsgas während des Sterilisationsprozesses unter Überdruck vor. Zum Ablaß des Gases erfolgt zunächst ein Öffnen der Ventile 7 und 8, so daß das Gas in Richtung der eingezeichneten Pfeile 9 und 11 durch die Leitung 12 strömt. Bei etwa Atmosphärendruck werden das Ventil 8 geschlossen und die Ventile 13 und 14 geöffnet. Dadurch kann ein weiteres Abpumpen bzw. Evakuieren der Kammer 1 mit Hilfe der Vakuumpumpe 15 erfolgen, so daß das abgepumpte Gas über die Vakuumpumpe 15 und die Leitung 16 in die Leitung 12 gefördert wird. Die Vakuumpumpe 15 ist zweckmäßigerweise als voreinlaßgekühlte zwei- oder dreistufige Roots- oder Wälzkolbenvakuumpumpe ausgebildet, so daß ein ölfreies Abpumpen der Gase sichergestellt ist. Der Rootspumpe ist ein wasserdurchströmter Kühler 17 nachgeschaltet, der eine unzulässige Erwärmung der Gase verhindert. In die Leitung 16 ist das Rückschlagventil 18 eingeschaltet, das ein Eindringen der Gase in die Leitung 16 während der Überdruckphase verhindert. Nach dem Erreichen eines Druckes von etwa 70 mbar (bei zweistufiger Roots- bzw. Wälzkolbenvakuumpumpe) bzw. 10 mbar (bei dreistufiger Pumpe) werden zum einen das Ventil 7 geschlossen, das Ventil 19 geöffnet und die weitere Vakuumpumpe 21 eingeschaltet. Dadurch wird die Kammer 1 auf einen Enddruck von etwa $10^{-2}$ mbar evakuiert. Das Restgas wird ins Freie abgeleitet. Durch diese

0130319

Restevakuierung entsteht nur ein geringer Verlust. Für eine Rückgewinnung dieses geringen Anteils wäre ein unverhältnismäßig hoher Aufwand erforderlich.

Beim Ausführungsbeispiel nach Fig. 1 gelangt das zu reinigende Äthylenoxid durch die Leitung 12 mit dem Rückschlagventil 22 in einen generell mit 23 bezeichneten Adsorber, in dem die Trocknung des Äthylenoxids erfolgt. In der Leitung 12 ist noch ein Filter 10 oder eine ähnliche Einrichtung vorgesehen. Sie dient der Entfernung mechanischer Verunreinigungen (Staub oder dergleichen) aus dem Gas. Der Adsorber 23 umfaßt zwei wechselweise betriebene Behälter 24 und 25, die jeweils mit einer elektrischen Mantelheizung 26 bzw. 27 ausgerüstet sind. Die beiden Behälter 24 und 25 sind mit Molekularsieb, Silicagel oder Alugel gefüllt. Je nach Stellung der Dreiwege-Ventile 28 und 29 strömt das Gas bei etwa Atmosphärendruck durch einen der beiden Behälter 24 und 25 und gelangt in die Leitung 31 mit dem Kompressor 32 und dem nachgeschalteten Kühler 33. Mit Hilfe des Kompressors 32 und des Kondensators 33 wird das Äthylenoxid auf einige Bar (2 bis 6 bar) verdichtet und auf 10 bis 40$^{\circ}$ C abgekühlt. Die Folge ist eine Verflüssigung des Äthylenoxids, das sich im Druckbehälter 34 sammelt. Die im Gasgemisch mitgeführten gasförmigen Verunreinigungen (Luft, Stickstoff und dergleichen) sammeln sich als nichtkondensierbare Bestandteile über dem flüssigen Äthylenoxid im Druckbehälter 34 an. Über die Abgasleitung 35 mit dem Ventil 36 werden sie zeitweise abgelassen. Die Abgasleitung 35 mündet in eine Hauptabgasleitung 40.

Das flüssige gerinigte Äthylenoxid wird über die Leitung 37 mit den Ventilen 39 und 41 der Sterilisationskammer 1 wieder zugeführt. Im Verdampfer 42 erfolgt der Übergang von der Flüssig- in die Gasphase. An die Leitung 37 ist noch über das Ventil 43 ein Äthylenoxid-Vorratsbehälter 44 angeschlossen, mit dem Äthylenoxid-Verluste ausgeglichen werden können.

Das Ausführungsbeispiel nach Fig. 2 dient der Reinigung eines aus Äthylenoxid und Dichlordifluormethan bestehenden Gemisches. Die Art und Weise der Trocknung, Verflüssigung und Wiederzuführung zur Sterilisationskammer 1 ist identisch mit Fig. 1. Unterschiedlich gegenüber Fig. 1 ist, daß dem Adsorber 23 ein Pufferbehälter 51 vorgeschaltet ist. Diesem wiederum sind in der Leitung 12 ein Kompressor 52 und ein Kühler 53 vorgeschaltet. Hiermit erfolgt ein Druckaufbau im Behälter 51 auf etwa 5 bar. Aus dem Pufferbehälter gelangt das Gasgemisch bei dem erhöhten Druck in den bereits beschriebenen Adsorber 23 und - nach der Trocknung - zum Kompressor 32. Mittels dieses Kompressors wird das Gasgemisch auf ca. 16 bis 20 bar komprimiert, im Kühler 33 auf ca. $20^{\circ}$ C abgekühlt und dadurch verflüssigt. Die Pufferung des Gases im Behälter 51 hat den Vorteil eines flexibleren Betriebs der Anlage.

Die beiden Adsorberbehälter 24 und 25 des Adsorbers 23 werden wechselweise betrieben. Während der eine der beiden Behälter in Betrieb ist, erfolgt die Regenerierung des Adsorbermaterials des anderen Behälters mit reinem Stickstoff. Dieses wird aus dem Vorrat 55 über die Leitung 56 und das Dreiwegeventil 57 dem jeweils zu regenerierenden Adsorber zugeführt. Über die Ventile 58 und 59 verläßt das der Regenerierung dienende Gas den jeweiligen Adsorber und wird der Hauptabgasleitung 40 zugeführt. Während der Regenerierung wird der jeweilige Behälter mittels der Mantelheizung auf ca. $250^{\circ}$ C aufgeheizt. Zweckmäßig ist es, den jeweils zu regenerierenden Behälter nach der eigentlichen Regenerierung unter Aufrechterhaltung der Beheizung zu evakuieren. Dazu ist an die Leitung 56 über das Ventil 62 und den Kühler 63 die Vakuumpumpe 64 angeschlossen. Diese Vakuumpumpe 64, die Pumpe 21 und auch eine weitere nicht dargestellte Vakuumpumpe, die der Evakuierung des Gesamtsystems vor der Inbetriebnahme dient, können ölgedichtete Pumpen sein, da sie mit dem Sterilisationsgas nicht unmittelbar in Berührung kommen.

Die beiden Kompressoren 32 und 52 sind als ölfrei arbeitende Kompressoren - vorzugsweise als Rootsgebläse - ausgebildet. Dadurch ist eine Kontamination der Gase mit Öl vermieden.

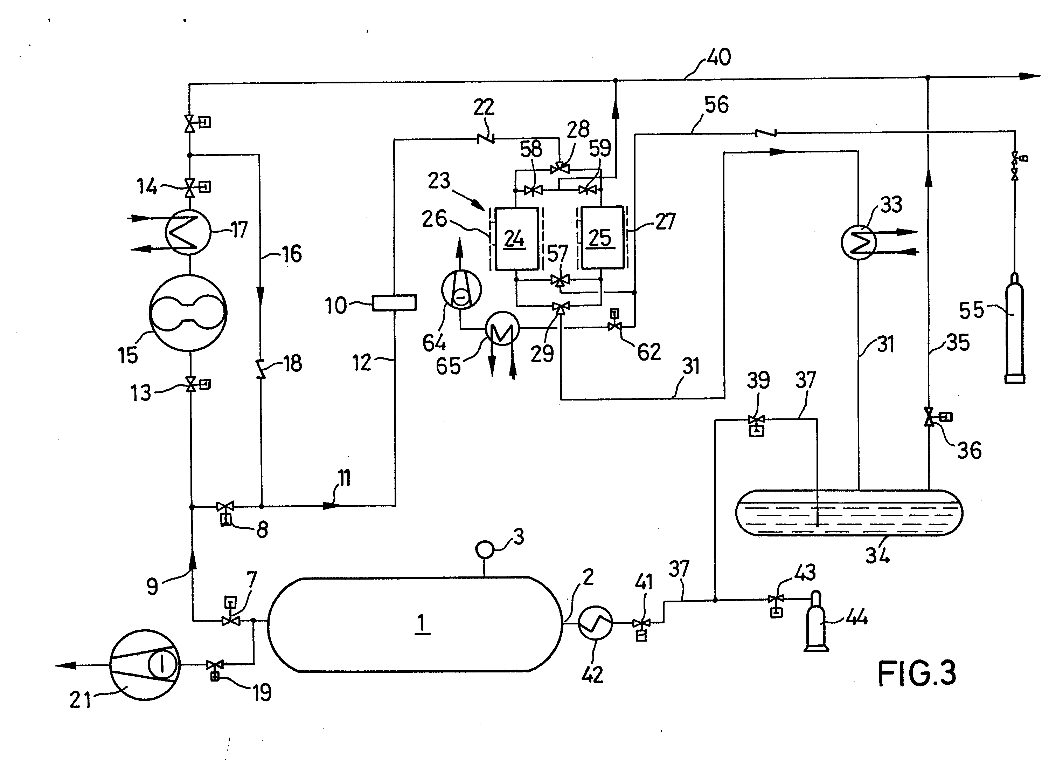

Das Ausführungsbeispiel nach Fig. 3 entspricht weitgehend dem Ausführungsbeispiel nach Fig. 1. Unterschiedlich ist, daß die Verflüssigung durch Temperaturerniedrigung erreicht wird. Der Kompressor 32 ist deshalb entfallen. Statt dessen ist die Kühleinrichtung 33 so gestaltet, daß die erforderlichen tiefen Temperaturen erzielt werden. Mit Hilfe von Kältesolen lassen sich z. B. Temperaturen bis zu $-100^{\circ}$ C, mit Hilfe von verdampfendem Stickstoff bis zu $-190^{\circ}$ C erzielen. Im letzteren Fall kann die geregelte Beimischung von warmem Stickstoff dazu dienen, höhere Verflüssigungstemperaturen einzustellen und einzuhalten.

Der Adsorptionszyklus wird mit Hilfe einer Zeituhr gesteuert, z. B. im 8-Stundenrhythmus. Diese Zeituhr steuert u. a. die Ventile 28, 29 sowie 57 bis 59 und 62 derart, daß sich stets ein Adsorberbehälter in der Betriebsphase und der jeweils andere Adsorberbehälter in der Regenerationsphase befinden. Auch die Mantelheizungen 26 und 27 sowie die Vakuumpumpe 64 können mittels der Zeituhr gesteuert werden.

Die zu allen Ausführungsbeispielen beschriebene Trocknungseinrichtung besteht aus einem Adsorber 23 mit zwei Betten 24 und 25. Stattdessen können auch andere Trockner vorgesehen sein, in denen das Gas oberhalb seiner Verflüssigungstemperatur getrocknet wird. Desweiteren kann die Trockeneinrichtung der Sterilisationskammer 1 unmittelbar nachgeordnet, d. h. auf der Saugseite der Vakuumpumpe 15 angeordnet sein. Die Trockeneinrichtung kann z. B. ein regenerierbarer Kältetrockner sein.

Die Kompressoren 32 (Fig. 1) bzw. 32 und 52 (Fig. 2) werden druckabhängig ein- und ausgeschaltet. Die Trocknung des Äthylenoxids (Fig. 1) erfolgt bei 1 bis 1,2 bar, so daß vor dem Adsorber 23 ein Kompressor nicht erforderlich ist. Danach erfolgt die Druckerhöhung auf 2 bis 6 bar zum Zweck der Verflüssigung mit Hilfe des Kompressors 32. Bei den genannten Druckwerten schaltet der Kompressor 32 ein bzw. aus.

Die Trocknung des Äthylenoxid-Frigen-Gemisches (Fig. 2) erfolgt bei ca. 1 bis 5 bar. Bei diesen Druckwerten wird Kompressor 52 ein- bzw. ausgeschaltet. Zur nachfolgenden Verflüssigung ist eine Druckerhöhung auf 5 bis 20 bar mit Hilfe des Kompressors 32 erforderlich. Die den Kompressoren jeweils nachgeschalteten Kühler verhindern eine allzu starke Erwärmung der geförderten Gase.

Zur Verflüssigung der Sterilisationsgase können auch ölfrei arbeitende ein- oder zweistufige Metallmembran- oder Kolbenverdichter eingesetzt werden. Für die Verflüssigung des Äthylenoxids bei 5 bis 6 bar (Fig. 1) reicht ein einstufiger Verdichter dieser Art aus. Bei der Verflüssigung des Sterilisationsgasgemisches durch Druckerhöhung auf bis zu 20 bar ist es zweckmäßig, einen zweistufigen Verdichter zu verwenden, das Gas mit der ersten Stufe auf 4 bis 5 bar zu verdichten und die Kühlung des Gases nach der ersten Stufe vorzunehmen.

- 8 -

Verfahren und Vorrichtung zur Reinigung von Äthylenoxid oder einem Gemisch aus Äthylenoxid und einem anwendungsspezifischen Zusatz

ANSPRÜCHE

1. Verfahren zur Reinigung von Äthylenoxid oder einem Gemisch aus Äthylenoxid und einem anwendungsspezifischen Zusatz, d a d u r c h   g e k e n n z e i c h n e t , daß das Gas getrocknet und bei einem oberhalb des Verflüssigungsdruckes von Luft liegenden Druck verflüssigt wird.

2. Verfahren nach Anspruch 1, d a d u r c h   g e k e n n z e i c h n e t , daß das Gas in einem Adsorber (23) mit Molekularsieb, Silicagel, Alugel oder dergleichen getrocknet wird.

3. Verfahren nach Anspruch 1 oder 2, d a d u r c h   g e k e n n z e i c h n e t , daß die Verflüssigung durch Druckerhöhung und/oder durch Temperaturerniedrigung erzielt wird.

4. Verfahren nach Anspruch 1, 2 oder 3, d a d u r c h   g e k e n n z e i c h n e t , daß bei Temperaturen im Zimmertemperaturbereich der Verflüssigungsdruck bei reinem Äthylenoxid ca. 1,5 bar und bei dem Gemisch von Äthylenoxid und fluoriertem Chlorkohlenwasserstoff oder einem anderen anwendungsspezifischen Zusatz ca. 5 bar überschreitet.

0130319

5. Verfahren nach Anspruch 4, d a d u r c h g e k e n n z e i c h n e t , daß das Gemisch aus Äthylenoxid und fluoriertem Chlorkohlenwasserstoff vor der Trocknung in einem Speicherbehälter (51) bei einem Druck von bis zu 6 bar gepuffert wird.

6. Verfahren nach Anspruch 1, 2 oder 3, d a d u r c h g e k e n n z e i c h n e t , daß bei Drücken im Atmosphärendruckbereich die Temperaturen unterhalb von 20$^{\circ}$ C (bei reinem Äthylenoxid) bzw. -30$^{\circ}$ C (bei Gemischen) liegen.

7. Verfahren zum Betrieb einer Sterilisationsanlage mit einer Sterilisationskammer, d a d u r c h g e k e n n z e i c h n e t , daß die Sterilisationskammer (1) nach einem Sterilisationsprozeß druckentlastet und danach bis auf einen Druck zwischen 10 und 100 mbar mit Hilfe einer ölfrei arbeitenden Vakuumpumpe evakuiert wird und daß die abgelassenen und die abgesaugten Gase nach einem Verfahren nach den vorhergehenden Ansprüchen gereinigt und zurückgewonnen werden.

8. Vorrichtung zur Durchführung eines der Verfahren nach den Ansprüchen 1 bis 7, g e k e n n z e i c h n e t durch mindestens einen Adsorber (23) zur Trocknung des Gases sowie durch mindestens einen Kompressor (32) zur Verflüssigung des Gases durch Druckerhöhung.

9. Vorrichtung nach Anspruch 8, d a d u r c h g e k e n n z e i c h n e t , daß die das Sterilisationsgas fördernden Kompressoren (32, 52) und Vakuumpumpen (15) nach dem Roots- oder Schraubenprinzip ölfrei arbeitende Maschinen sind.

10. Vorrichtung nach Anspruch 8, d a d u r c h g e k e n n z e i c h n e t , daß die Kompressoren von ölfrei arbeitenden Metallmembran- oder Kolbenverdichtern gebildet werden.

11. Vorrichtung nach Anspruch 9 oder 10, d a d u r c h g e k e n n z e i c h n e t , daß den Kompressoren (32, 52) und Vakuumpumpen (15) jeweils ein Kühler (17, 33, 53) nachgeschaltet ist.

12. Vorrichtung nach Anspruch 11 zur Durchführung eines Verfahrens nach Anspruch 3 oder 4, d a d u r c h g e k e n n z e i c h n e t , daß ein zweistufig arbeitender Kompressor mit Zwischenkühlung verwendet wird.

13. Vorrichtung nach Anspruch 8, 9 oder 10, d a d u r c h g e k e n n z e i c h n e t , daß dem Adsorber (23) ein Pufferbehälter (51) vorgelagert ist.

14. Vorrichtung zur Durchführung eines der Verfahren nach den Ansprüchen 1 bis 7, g e k e n n z e i c h n e t durch mindestens einen Adsorber (23) zur Trocknung des Gases sowie durch mindestens eine Kühleinrichtung (33) zur Verflüssigung des Gases durch Temperaturerniedrigung.

FIG.1

0130319

FIG.2

FIG.3

-3/3-

0130319